# EUROPEAN PATENT APPLICATION

(11) **EP 4 356 974 A1**
(43) Date of publication of application: **24.04.2024**
(21) Application number: 22383019.1
(22) Date of filing: 21.10.2022
(51) Int. Cl.: A61P 37/02, C07C 59/265, C07H 3/02, A61K 31/7004, A61K 31/194

(54) **COMBINATION OF LACTOSE AND CITRIC ACID USEFUL TO MODULATE THE IMMUNE SYSTEM**

(71) Applicant: Campos Vidal, Fernando, 07180 Santa Ponca (ES)
(72) Inventor: Campos Vidal, Fernando, 07180 Santa Ponca (ES)
(74) Representative: ZBM Patents - Zea, Barlocci & Markvardsen

(57) **Abstract**

A product which comprises a crystallized mixture of two active ingredients, wherein the crystallized mixture is a mixture in which the two active ingredients have crystallized together; and wherein the active ingredients are: a) lactose or a pharmaceutically acceptable salt thereof, and b) citric acid or a pharmaceutically acceptable salt thereof, in a weight ratio in the crystallized mixture from 89:11 to 69:31 expressed with respect to lactose:citric acid in free form; pharmaceutical compositions comprising it and their use in the prevention and/or treatment of a disease or condition through the modulation of the immune system.

## Description

### Technical Field

The invention relates to the field of pharmacy, in particular, it relates to a product with immunomodulatory properties, pharmaceutical compositions comprising them, and their use to modulate the immune system for the treatment of several diseases.

### Background Art

Viral diseases are one of the health care problems with the greatest socioeconomic impact. Viral conditions (e.g., cold, flu) account for 30-40% of emergency consultations in health care centres and often progress to secondary bacterial infections which, in high-risk patients, can sometimes lead to sometimes fatal illnesses (e.g., pneumonia).

Respiratory diseases of viral etiology are one of the major failures of modern medicine and pharmacology. Flu and colds are by far the most common infectious diseases in the world. Each person has an average of 2.5 colds a year, flu every 5-10 years. The global population is 7,432 million, this means that every year give: 18,580 million colds cases 1,486 / 743.2 million flu cases. Cold and flu cause discomfort, loss of scholar days, loss of working days, pharmaceutical spending, hospital admissions and sometimes complications that can lead to death.

No cure for the common cold exists, but the symptoms can be treated. Therefore, the treatment offers symptomatic relief. No medications or herbal remedies have been conclusively demonstrated to shorten the duration of infection. There is no vaccine for the common cold. Vaccination has proved difficult, as there are many viruses involved and they mutate rapidly. Creation of a broadly effective vaccine, is thus, highly improbable.

No cure for flu exists either, but the symptoms can be treated. Therefore, the treatment offers symptomatic relief. Although there is a specific antiviral treatment for flu, these are expensive, not very effective and often have unwanted side effects. The influenza vaccine is recommended for high-risk groups. In healthy adults, it is modestly effective in decreasing the amount of influenza symptoms in population.

Flu and colds are not only an individual pathology; they also have great social significance and constitute a big headache for those who manage the budgets of health services. Both produce high economic losses, and in the case of influenza, due to its marked seasonal nature, it causes hospital collapse, and this can generate enormous problems. In large part due to the high attack rate, colds and influenza impose a greater economic burden than many other clinical conditions. The pending availability of effective antiviral therapies for these common and expensive diseases warrants further attention to the development of these types of therapies.

Cold and flu Drugs are relatively effective and safe drugs which can be bought at pharmacies and local stores, over the counter without a doctor's prescription.
These drugs are seldom used for common ailments. It may contain decongestants, pain relievers, antihistamines, and cough suppressants. They are dispensed by the pharmacists after the verification of customer's age and identification. Acetaminophen, Aspirin, and Ibuprofen are the most common over the counter (OTC) drugs that do not require prescription from healthcare professionals for selling it to consumers. However, The actual used drugs only provide symptomatic relief, but they do not shorten the duration of the disease. Cold lasts for 8-10 days. Flu lasts for 7 days, 15 days until full recovery. Thus, there is still the need for people of shorten the duration of colds and flu.

A great effort is being made to investigate new therapeutic strategies. For example, interferon (IFN), a product obtained through biotechnological processes, exerts a natural antiviral effect, but its usefulness as such when administered exogenously is lower than expected.

In recent years, immunotherapy has been developed as a promising therapy in form of complementary treatment for the diseases caused by viral infections. Attacks of microorganisms including viruses and bacteria can be counteracted with an efficient immune system and therefore, stimulation of body's defence mechanism against infections have been proven to be an effective approach.

Alhazmi et al., summarizes some of the efforts of using medicinal plants in "Medicinal Plants and Isolated Molecules Demonstrating Immunomodulation Activity as Potential Alternative Therapies for Viral Diseases Including COVID-19 Natural Immunomodulators for Viral Diseases" 2021, vol.12 (https://doi.org/10.3389/fimmu.2021.637553). For instance, a wide range of plant extracts, herbal products, and pure plant constituents have shown potential immunomodulation effects and used either as prophylactic or therapeutic agents for infectious diseases. Apart from infectious diseases, plant-based immunomodulators have been described as products that can help to treat several other immunologic and inflammatory illnesses including cancer, rheumatoid arthritis, plaque psoriasis, Crohn's disease, etc. These plant products and their bioactive molecules modulate immune responses through the stimulation and modification of lymphocytes, macrophages, and cytokine production. Many plant extracts contain a number of active principles, including polysaccharides, terpenoids, alkaloids, flavonoids, glycosides, and essential oils, which have the capability to maintain and/or stimulate the immune system primarily through the modulation of nonspecific immune responses. However, these plant extracts may contain one or more of the active constituents and their relative contribution and mechanism in the generation of immunomodulation activity are difficult to understand.

On the other hand, dietary supplements such as vitamins C, D and E are known to improve the immunity. The duration and severity of common cold has been shown to be reduced by a high dose of vitamin C and D supplements in patients. Vitamin E supplementation was found to improve cellular and humoral immunity and provides protection against infections; although, the degree of resistance is unclear.

Also, a large number of phytopharmaceuticals have been investigated for their beneficial effects in several diseases and many of them exerted significant immunomodulation properties. For example, resveratrol, an active constituent from grapevine, red wine, and peanuts, Epigallocatechin-3-gallate (EGCG), a polyphenol constituent from green tea, alkylamides, or polysaccharide constituents.

T Nagai et al, in "In vivo anti-influenza virus activity of Kampo (Japanese herbal) medicine "Sho-Seiryu-to -stimulation of mucosal immune system and effect on allergic pulmonary inflammation model mice" Immunopharmacology and Immunotoxicology, 1998, vol. 20, pp. 267-281 discloses the use of Kampo for both prophylaxis and treatment of influenza virus infection on patients with allergic pulmonary inflammation, such as bronchial asthma.

Finally, α--lactose has previously been described as a promising immunomodulatory agent in the treatment of sepsis (see Yao et al.; "a-Lactose Improves the Survival of Septic Mice by Blockade of TIM-3 Signaling to Prevent NKT Cell Apoptosis and Attenuate Cytokine Storm"; Shock (Augusta, Ga. 2017, vol. 47(3), pp. 337-345).

Also, R. Chowdhury et al.; in " Effect of citric acid, avilamycin, and their combination on the performance, tibia ash, and immune status of broilers." Poult. Sci., 2009, vol. 88, pp. 1616-1622 discloses that citric acid as a feed additive produced healthy broilers that possessed a higher immune status to fight against enteric pathogens and infectious disease. It is also known that chicks fed acidified diets had better immune response as indicated by their higher serum globulin and relative lymphoid organs than the control (see Abdel-Fattah, S. A. et al., "Thyroid activity of broiler chicks fed supplemental organic acids.", Int. J. Poult. Sci, 2008, vol. 7, pp. 215-222).

However, from what is known in the art, there is still the need of finding effective treatments to treat viral diseases.

### Summary of Invention

The inventor of the present invention has found that a combination of lactose and citric acid when combined in certain ratios and conditions give rise to the appearance of improved immunomodulatory properties in the product with respect to the precursor molecules alone. It shows an Immunomodulatory effect (stimulates or depresses the immune system), its character is non-specific since it affects the whole immune system; it also has anti-inflammatory effect; and it also shows the effect of improving physical performance on short-term efforts. The inventor has found that the antiviral capacity of the combination of the invention due to its stimulating effect of the immune system is unexpectedly higher than when using the components alone.

Advantageously, this combination of lactose and citric acid in specific ratios is able to achieve the total disappearance of all cold symptoms in an average of just 24 hours and flu symptoms in 48 hours, providing a dramatic shortening in the intensity and duration of disease.

Furthermore, the data of the examples of the present invention illustrates that it is able to treat several viral diseases, in particular, it is especially useful for the treatment of: influenza, common cold, herpes simplex, herpes zoster, plantar papilloma, warts, stomach flu, viral pneumonia, mononucleosis, viral conjunctivitis, mumps, cytomegalovirus, varicella, smallpox, respiratory syncytial virus, and cancer. Further advantages are that it offers a simple, safe, effective and cheap formulation.

Finally, the use of the product of the present invention in the recommended amounts does not cause any type of side effect and as a general rule it is compatible with the use of other medicines. Neither it presents any incompatibilities with regard to the patient's age, sex or belonging to a specific ethnic group.

Accordingly, a first aspect of the present invention relates to a product which comprises a crystallized mixture of two active ingredients, wherein the crystallized mixture is a mixture in which the two active ingredients have crystallized together; and wherein the active ingredients are: a) lactose or a pharmaceutically acceptable salt thereof, and b) citric acid or a pharmaceutically acceptable salt thereof, in a weight ratio in the crystallized mixture from 89:11 to 69:31 expressed with respect to lactose:citric acid in free form.

A second aspect of the present invention relates to a process for preparing the product as defined above, comprising the steps of:
a) mixing the lactose or the pharmaceutically acceptable salt thereof in water at an appropriate temperature to form a solution; b) mixing the citric acid or the pharmaceutically acceptable salt thereof in water at an appropriate temperature to form a solution; c) mixing the solution a) with the solution b) in a proportion such that the weight ratio lactose: citric acid is from 89:11 to 69:31; and d) isolating the compound thus obtained.

A third aspect of the present invention relates to a pharmaceutical composition comprising a therapeutically effective amount of the product as defined above, together with appropriate pharmaceutically excipients and/or carriers.

A fourth aspect of the present invention relates to a food supplement comprising an immunomodulatory effective amount of the product as defined above.

A fifth aspect of the present invention relates to a product as defined above, for use as a medicament.

A sixth aspect of the present invention relates to a product as defined above, for use for modulating the immune system.

Finally, a seven aspect of the present invention is the use of the product as defined above, for improving the discomfort of premenstrual syndrome or for improving physical performance.

### Brief Description of Drawings

FIG.1 shows the X-ray diffractogram of the product of the invention comprising lactose citric acid in a weight ratio 73:23 in the crystallized mixture.
FIG. 2 shows the crystal form of the combination under an optical microscope 40X.
FIG. 3 Shows cold symptoms.
FIG. 4 shows influenza virus infection timeline.
FIG. 5 shows the results of the formulation to treat virus herpes simplex 1 (2 x 103 p.f.u./mice). p.f.u. means plaque-forming units which are the number of virus particles capable of forming plaques per unit of volume. The line with squares represents mice without treatment, the line with triangles represents the mice with the treatment with the product according to the invention from the beginning of the infection, the line with circles represents the mice with the treatment with the product according to the invention from 2 days before the infection.
FIG. 6 shows the results of the cytokines release assay. Cpd X corresponds to the product of the present invention (corresponding to that of Example 1).
FIG. 7 shows the tumour progression after administering the formulation of the present invention in a dog suffering a breast tumour.
FIG. 8 shows the result after administering the formulation of the present invention in basal cell carcinoma in a human. A) is a picture taken 12.09.2015, one day before starting the treatment, and B) is a picture taken 2712/2015, nearly 3 months after the treatment.
FIG. 9 shows the clinical evolution of dogs affected by canine parvovirus.
FIG. 10 shows survival rate of treated dogs suffering from canine parvovirus.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

As used herein, the indefinite articles "a" and "an" are synonymous with "at least one" or "one or more." Unless indicated otherwise, definite articles used herein, such as "the" also include the plural of the noun.

As mentioned above, it is part of the invention a product which comprises a crystallized mixture of two active ingredients, wherein the crystallized mixture is a mixture in which the two active ingredients have crystallized together; and wherein the active ingredients are: a) lactose or a pharmaceutically acceptable salt thereof, and b) citric acid or a pharmaceutically acceptable salt thereof, in a weight ratio in the crystallized mixture from 89:11 to 69:31 expressed with respect to lactose:citric acid in free form. The molar ratio has been determined by 1H NMR in the conditions specified in the examples.

By the term crystallized mixture is understood a mixture that is obtainable by crystallization from a solution of the components of the mixture: The term "crystallization" is understood as the physical transformation of a liquid or solution to a crystal, which is a solid with an ordered internal arrangement of molecules, ions, or atoms. In the present case, the solution comprises both active ingredients, lactose or a pharmaceutically acceptable salt thereof, and citric acid or a pharmaceutically acceptable salt thereof, which are dissolved, and this solution is transformed into a solid comprising crystals of both active ingredients, and encompassing any crystallized form of the active ingredients. Thus, for the transformation from liquid to solid, both products crystallize from the same solution.

Lactose is a disaccharide, more precisely, a sugar composed of galactose and glucose subunits and has the formula below:

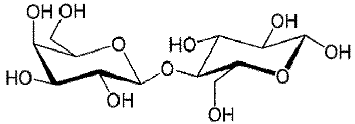

Citric acid is an organic compound having the formula below:

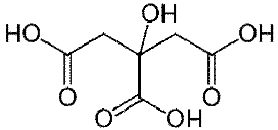

In a particular embodiment, the product of the present invention is that in which the mixture comprises citric acid in free form. In another particular embodiment, the product of the present invention is that in which the mixture comprises lactose in free form. In another particular embodiment, the product of the present invention is that in which the mixture comprises lactose and citric acid in free form. Both may be in the form of hydrates.

In another particular embodiment, the product of the present invention is that where the weight ratio lactose:citric acid is from 86:14 to 70:30. In another particular embodiment, the product of the present invention is that where the weight ratio lactose:citric acid is from 80:20 to 70:30. In another particular embodiment, the product of the present invention is that where the weight ratio lactose:citric acid in the crystallized mixture is 77:23.

In another particular embodiment, either alone or in combination with any of the particular embodiments of the present invention, the product of the present invention is that where the lactose may be a mixture of alpha/beta lactose, generally the weight ratios of alpha/beta lactose is from 100:0 to 38:62. The alpha/beta ratio has been determined by ¹H NMR in the conditions specified in the examples.

In another particular embodiment of the invention, the product is that which consist of: a) lactose or a pharmaceutically acceptable salt thereof, and b) citric acid or a pharmaceutically acceptable salt thereof, in a weight ratio in the crystallized mixture from 89:11 to 69:31 expressed with respect to lactose:citric acid in free form, more particularly, in in a weight ratio from 86:14 to 70:30, and even more particularly in a weight ratio 77:23.

In another particular embodiment, either alone or in combination with any of the particular embodiments of the present invention, the product of the present invention is characterized by having an X-ray diffractogram that comprises characteristic peaks at approximately 12.5, 16.4, 19.1, 19.6, 20.9, 21.2, 23.8 degrees 2 theta at a Cu-K_{α}1 radiation =1,54060 Å. In another particular embodiment the X-ray diffractogram further comprises the following peaks at approximately 15.1, 18.2, 22.8 degrees 2 theta at a Cu-K_{α}1 radiation =1,54060 Å. The X-ray diffractogram is obtained using BRUKER D8 ADVANCE diffractometer at a Cu-K_{α}1 radiation (λ= 1,54060 A) and at a power of 40 kV - 40x mA. In a particular embodiment, the product of the present invention is characterized by having the peaks of the X-ray included in the following table:

| Angle 2θ (°) | d Value (Å) | Net Intensity | Rel. Intensity |
|---|---|---|---|
| 8.2 | 10.8 | 1185 | 0.01 |
| 11.8 | 7.5 | 2003 | 0.02 |
| 12.5 | 7.1 | 34674 | 0.43 |
| 14.4 | 6.1 | 9381 | 0.01 |
| 15 | 5.9 | 12450 | 0.03 |
| 15.1 | 5.8 | 10333 | 0.13 |
| 16.4 | 5.4 | 27238 | 0.34 |
| 17 | 5.2 | 6237 | 0.08 |
| 18 | 4.9 | 2642 | 0.03 |
| 18.2 | 4.9 | 9188 | 0.2 |
| 19.1 | 4.6 | 34986 | 0.4 |
| 19.6 | 4.5 | 41242 | 0.52 |
| 20 | 4.4 | 79473 | 1 |
| 20.9 | 4.2 | 13272 | 0.17 |
| 21.2 | 4.2 | 24561 | 0.31 |
| 22.3 | 4 | 7480 | 0.09 |
| 22.8 | 3.9 | 8243 | 0.10 |
| 23 | 3.8 | 7927 | 0.1 |
| 23.8 | 3.7 | 15661 | 0.2 |
| 24.4 | 3.6 | 1740 | 0.02 |
| 25.2 | 3.5 | 2193 | 0.03 |
| 25.6 | 3.5 | 7569 | 0.09 |
| 26.2 | 3.4 | 2167 | 0.,03 |
| 26.5 | 3.4 | 965 | 0.01 |
| 26.9 | 3.3 | 975 | 0.02 |
| 27.5 | 3.2 | 6481 | 0.08 |
| 28.2 | 3.1 | 3431 | 0.04 |
| 28.,5 | 3,13 | 3057 | 0.04 |
| 28.8 | 3.1 | 1483 | 0.02 |
| 29.1 | 3.07 | 1284 | 0.02 |
| 29,3 | 3,04 | 2878 | 0.04 |
| 29,3 | 3 | 2874 | 0.04 |
| 30.7 | 2.9 | 1742 | 0.02 |
| 31 | 2.87 | 3367 | 0.04 |
| 31.5 | 2.8 | 2279 | 0.03 |
| 33,2 | 2,7 | 4207 | 0.05 |
| 34.6 | 2.6 | 3562 | 0.05 |
| 34.9 | 2.56 | 4444 | 0.06 |
| 35.3 | 2.53 | 1560 | 0,02 |
| 35.6 | 2.52 | 1796 | 0.02 |
| 36.2 | 2.48 | 3790 | 0.05 |
| 36.9 | 2.43 | 6004 | 0.07 |
| 37.6 | 2.39 | 7660 | 0.1 |
| 38.2 | 2.35 | 5043 | 0.06 |
| 38.6 | 2.33 | 4928 | 0,06 |
| 39 | 2.3 | 926 | 0.01 |
| 39.4 | 2.28 | 1907 | 0.02 |
| 39.9 | 2.26 | 2678 | 0.03 |
| 40.7 | 2.2 | 2202 | 0.03 |
| 41,8 | 2.16 | 2113 | 0.03 |
| 42.4 | 2.13 | 1738 | 0.02 |
| 42,6 | 2.12 | 1114 | 0.01 |
| 43.1 | 2.1 | 1781 | 0.02 |
| 44.3 | 2.04 | 800 | 0.01 |
| 45.7 | 1.99 | 1278 | 0.02 |
| 47.3 | 1.92 | 1282 | 0.02 |
| 47.,8 | 1,9 | 1612 | 0.02 |
| 48.9 | 186 | 804 | 0.01 |
| 50.3 | 1.84 | 1464 | 0.03 |
| 51.9 | 1.76 | 1486 | 0.,02 |

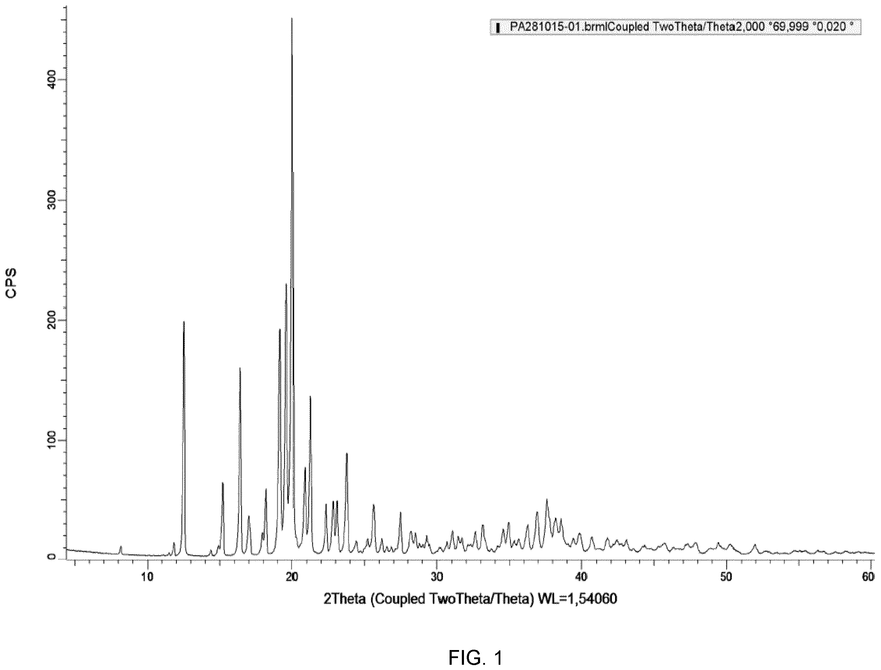

In a particular embodiment the product of the present invention is characterized by the X-ray of FIG. 1.

The preparation of the combination of lactose and citric acid according to the invention may be carried out starting from the two precursor molecules, lactose and acid citric, or their respective salts, which are separately diluted in water at different temperatures and subsequently under certain conditions, are mixed by shaking, in certain proportions. The resulting mixture may be dried and passed to storage.

The product of the present invention may be prepared by a process comprising the steps of a) mixing the lactose or the pharmaceutically acceptable salt thereof in water at an appropriate temperature to form a solution; b) mixing the citric acid or the pharmaceutically acceptable salt thereof in water at an appropriate temperature to form a solution; c) mixing the solution a) with the solution b) at an appropriate temperature in a proportion such that the weight ratio lactose citric acid is from 89:11 to 69:31; and d) isolating the compound thus obtained.

The most adequate conditions for carrying out said processes vary depending on the parameters considered by the expert in the art, such as, for example, the concentration of the starting material, temperature, and the like. These can be easily determined by said skilled person in the art by routine tests and with the help of the teachings of the examples given in this description.

In a particular embodiment, the water used is deionized water.

In a particular embodiment, the temperature at which the lactose or the pharmaceutically acceptable salt thereof is mixed with water is in a range from 15°C to 100 °C. In another particular embodiment, the temperature at which the lactose or the pharmaceutically acceptable salt thereof is mixed with water is in a range from 15°C to 90 °C. In another particular embodiment, the temperature is in a range from 20 to 90 °C. In another particular embodiment, the temperature is in a range from 20 to 30 °C. In another particular embodiment, the temperature is room temperature (20-25 °C). The solubility of citric acid in water is 18.904 g/100 ml. The amount of water is at least an amount in which at the working temperature the lactose is dissolved. The amounts will vary depending on the temperature.

In a particular embodiment, the temperature at which the citric acid or the pharmaceutically acceptable salt thereof is mixed with water is in a range from 15°C to 100 °C. In another particular embodiment, the temperature at which the citric acid or the pharmaceutically acceptable salt thereof is mixed with water is in a range from 15°C to 90 °C. In another particular embodiment, the temperature is in a range from 20 to 90 °C. In another particular embodiment, the temperature is in a range from 20 to 30 °C. In another particular embodiment, the temperature is room temperature (20-25 °C). The solubility of citric acid in water is 133 g/100 ml at 22 °C. The amount of water is at least an amount in which at the working temperature the lactose is dissolved.

Both solutions can be mixed at a temperature which is a temperature in the range from 15 to 100 °C. The solutions can be mixed in any order. In a particular embodiment, the solution of citric acid or their salts is added over the solution of lactose or their salts.

When the solutions are mixed at a certain temperature, they can be cooled at room temperature favoring the crystallization. The crystallization also occurs when water is removed.

In a particular embodiment, the product of the present invention is isolated by filtration. In another particular embodiment, the product of the present invention is isolated by a process of removal of water, such as evaporation at ambient pressure or under vacuum. The mixture may be desiccated, generally up to a content of water equal to or lower than 8% by weight.

In particular, the crystallization of the lactose: citric acid mixture of the present invention gives rise to crystalline structures that are not an accumulation of the crystalline forms of lactose and citric acid separately. The crystallization process is greatly influenced by the drying temperature and drying time, giving rise to crystalline structures of very different appearance.

Thus, the product of the present invention may be a crystallized mixture in which the two active ingredients have crystallized together, comprising a) lactose or a pharmaceutically acceptable salt thereof, and b) citric acid or a pharmaceutically acceptable salt thereof, in a weight ratio with respect lactose:citric acid in free form in the crystallized mixture from 89:11 to 69:31.

The product of the present invention may also be defined by its preparation process. Thus, a crystallized mixture comprising a) lactose or a pharmaceutically acceptable salt thereof, and b) citric acid or a pharmaceutically acceptable salt thereof in a weight ratio with respect lactose:citric acid in free form from 89:11 to 69:31 obtainable by the process described above is also part of the present invention. All the particular embodiments of the product and of the process above or below are also particular embodiments when defining the product by its preparation process.

The storage does not require special temperature or conditioning. Preferably, the moisture of the molecule is kept below 8%. The moisture content may be equal to or below 7%, equal to or below 4%, equal to or below 2%, or even it can be anhydrous. The water content can be measured by known techniques, such as Karl-Fisher.

The preparation of pharmaceutically acceptable salts of the components as starting material of the product of the present invention can be carried out by methods known in the art. For instance, they can be prepared from the conjugate, which contains a basic or acidic moiety, by conventional chemical methods. Generally, such salts are, for example, prepared by reacting the free acid or base forms of these compounds with a stoichiometric amount of the appropriate pharmaceutically acceptable base or acid in water or in an organic solvent or in a mixture of them. Salts of lactose and of acid citric are also commercially available.

The product of the invention may be also characterized by its properties, in particular its stimulating effect of the immune system which is higher than when using the components alone.

The product of the present invention may be administered directly, i.e., without being mixed with further excipients. Alternatively, the product can be administered in the form of a pharmaceutical composition comprising a therapeutically effective amount of the product of the present invention as defined above, together with appropriate amounts of pharmaceutically acceptable carriers or excipients, thus, such pharmaceutical composition is also part of the invention.

Thus, the term "pharmaceutical composition" refers to a mixture of the product described herein with other chemical components, such as diluents or carriers. The pharmaceutical composition facilitates administration of the product to an organism.

The expression "therapeutically effective amount" as used herein, refers to the amount of a compound (the product of the present invention) that, when administered, is sufficient to prevent development of, or alleviate to some extent, one or more of the symptoms of the disease which is addressed. The particular dose of compound administered according to this invention will of course be determined by the particular circumstances surrounding the case, including the compound administered, the route of administration, the particular condition being treated, and the similar considerations.

The expression "pharmaceutically acceptable excipient, diluent or carrier" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and non-human animals without excessive toxicity, irritation, allergic response, immunogenicity or other problems or complications commensurate with a reasonable benefit/risk ratio.

The product of the present invention is very versatile, since it can be administered in different modes of administration such as oral, intravenous, intramuscular, or supra / Sublingual. In a particular embodiment, the pharmaceutical composition of the present invention is for oral administration.

In another particular embodiment, the pharmaceutical composition is in the form of capsules, tablets, soluble solutions, stick packs, among others. In another particular embodiment, the pharmaceutical composition is in the form of capsules. Advantageously, the small size of the capsules makes their administration very easy. The tablets may be for instance, chewable tablets, effervescent tablets, flash tablets, or coated tablets, in this case the coating may be aesthetic (to mask a bad taste for example), or functional (case of gastroresistant tablets, which cannot be crushed as they must pass unaltered until they reach the intestine). The tablets may also be slotted tablets, prepared for oral intake with water and which can be easily broken to adjust to a specific dose. The product may also be presented in the form of granules to be dissolved in water. Generally, kept inside sachets.

Examples of suitable pharmaceutically acceptable excipients for the pharmaceutical formulations according to the present invention are diluents, preservatives, solid binders, lubricants, surfactants, and the like. Except insofar as any conventional excipient medium is incompatible with a substance or its derivatives, such as by producing any undesirable biological effect or otherwise interacting in a deleterious manner with any other component(s) of the pharmaceutical composition, its use is contemplated to be within the scope of this invention.

Examples of diluents are lactose, starch, or calcium phosphate. Preservatives may be added to inhibit the growth of microorganisms. In case of using surfactants, generally, they are non-ionic and are used in very small quantities. Appropriate surfactants may be phospholipids such as lecithin, polysorbates such as Tween, sorbitan monooleates such as Span, polyoxyethylene ethers, or polyoxyethylenated castor oils.

The pharmaceutical composition may also include other components such as antioxidants, antiseptic agents capable of destroying pathogenic germs or opposing their proliferation outside or inside the organism, flavourings to mask or improve the organoleptic characteristics of taste and odor; colorants; sweeteners, thickeners, or stabilizers. Carboxymethyl cellulose can be used to facilitate dispersion.

Coating agents can form part of one or more layers of mixtures in the case of coated tablets, they can be natural or synthetic resins, gums, gelatine, inactive and insoluble fillers, sugars, plasticizers, polyols, waxes, colorants and, in some cases, flavoring agents. The relative amounts of the components of the product of the present invention, the pharmaceutically acceptable excipient, and/or any additional ingredients in a pharmaceutical composition of the invention will vary, depending upon the identity, size, and/or condition of the subject treated and further depending upon the route by which the composition is to be administered.

It is also part of the invention a food supplement comprising an immunomodulatory effective amount of the product as defined above.

The preclinical studies with the molecule show that both under infection and non-infection conditions, significantly stimulated the secretion of pro-inflammatory cytokines. The stimulation determines an increase in activity of the phagocytic cells of the immune system: macrophages, neutrophils, and dendritic cells. And also, of the different classes of T lymphocytes: cytotoxic T lymphocytes (or CD8 + lymphocytes), helper T lymphocytes (or CD4 + or helper lymphocytes), regulatory T lymphocytes, as well as NK cells. Its sequential and coordinated action explains the effects observed in the cure of different viral diseases. It influences the activity of the immune system in its unspecific response, stimulation the production of interferon y (+78.94%), reducing mobilization of neutrophils and holding constant the lymphocyte populations during infection. Advantageously, it does not induce the occurrence of adverse effects or clinically or pathologically.

As mentioned above, the product as defined above for use as a medicament is also part of the invention.

The term "medicament" as used herein is synonymous of a pharmaceutical or veterinary drug (also referred to as medicine, medication, or simply drug) use to cure, treat or prevent disease in animals, including humans, as widely accepted.

Also, the product as defined above for use for modulating the immune system in humans and animals, in particular mammals is part of the invention, as well as a product as defined above for use in the prevention and/or treatment of a disease or condition through the modulation of the immune system, wherein the disease or condition is selected from the group consisting of influenza, common cold, herpes simplex, herpes zoster, infections caused by the human papilloma virus, stomach flu, viral pneumonia, mononucleosis, viral conjunctivitis, mumps, cytomegalovirus, varicella, smallpox, respiratory syncytial virus, cancer, hepatitis B, hepatitis C, and chronic inflammation. This aspect can also be formulated as the use of product as defined above for the preparation of a medicament for the prevention and/or treatment of a disease or condition through the modulation of the immune system, wherein the disease or condition is selected from the group consisting of influenza, common cold, herpes simplex, herpes zoster, infections caused by the human papilloma virus, stomach flu, viral pneumonia, mononucleosis, viral conjunctivitis, mumps, cytomegalovirus, varicella, smallpox, respiratory syncytial virus, cancer, hepatitis B, hepatitis C, and chronic inflammation is also part of the invention. The invention also relates to a method of treatment of a mammal, including a human, suffering from or being susceptible of suffering from a disease or condition selected from the group consisting of influenza, common cold, herpes simplex, herpes zoster infections caused by the human papilloma virus, stomach flu, viral pneumonia, mononucleosis, viral conjunctivitis, mumps, cytomegalovirus, varicella, smallpox, respiratory syncytial virus, a cancer, hepatitis B, hepatitis C, and chronic inflammation, said method comprising the administration to said patient of a therapeutically effective amount of the product as defined above, together with pharmaceutically acceptable excipients or carriers.

In a particular embodiment, the infections caused by the human papilloma virus are selected from the group consisting of plantar papilloma, warts, genital papilloma in both men and women, anal papilloma, and cervical papilloma in women. Cervical papilloma in women often leads to cervical cancer. In another particular embodiment, the infections caused by the papilloma virus are selected from plantar papilloma and warts.

In a particular embodiment, the product as defined above is that wherein the disease or condition to treat is influenza or common cold. In another particular embodiment, either alone or in combination with any of the embodiments of the invention, the product as defined above is that where the treatment of influenza or common cold comprises starting it in the next 36 hours of appearing the symptoms of the disease. Unexpectedly, a much greater effectiveness, with a dramatic reduction in the intensity of symptoms and the duration of the disease in colds and flu is observed.

In another particular embodiment, either alone or in combination with any of the embodiments of the invention, the product as defined above is that wherein the disease or condition to treat is herpes.

In another particular embodiment, either alone or in combination with any of the embodiments of the invention, the product as defined above is that wherein the disease or condition to treat is cancer.

The product of the present invention is also advantageous since it does not have any type of known toxic effect in amounts that exceed dozens of times the lowest therapeutic dose. In the normal oral therapeutic dose, it is also very safe, admits doses higher than 50 times the normal dose, without producing any type of toxic effect.

Generally, the therapeutic dose is low, 9.4 mg/kg (+/-10%) of body weight, equivalent to 600 mg of active ingredient, orally, 3 times a day, for a n individual weighing 70 kilos. In severe or acute cases, the dose can be increased to a total of 8-10 daily doses. The amounts may vary depending on the illness or condition to be treated and the age and profile of the patient to be treated.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim and shall not be construed as limiting the scope of the claim. Furthermore, the present invention covers all possible combinations of particular and preferred embodiments described herein.

### Examples

Reference in examples to product according to the invention means a product approximately 73:23 weight ratio of lactose:citric acid in the crystallized mixture. The product is a crystallized mixture obtained as disclosed in Example 1.

The examples of the present invention were carried out with a product according to the invention comprising the product approximately 73:23 weight ratio of lactose:citric acid without further excipients.

### Example 1: Preparation process of the formulation under evaluation and characterization of the product

3 grams of commercial citric acid monohydrate were mixed with 6 grams of deionized water at room temperature. 17 grams of commercial lactose monohydrate was mixed with deionized water at 25°C, stirring energetically until the total dissolution of the lactose was achieved. Without letting the lactose solution cool, the mixture of citric acid and water was poured over it, stirring energetically. The chemical reaction that produces the formation of the characteristic glasses occurred instantaneously. The mixture was dried in an oven by subjecting it to a high temperature, 100°C-110°C until the amount of moisture in the mixture is a maximum of 8%, which ensures the preservation of the mixture at room temperature without the need for complementary measures.

¹H NMR spectra in D₂O at 25 °C that a mixture 73:23 weight ratio of lactose:citric acid in the crystallized mixture is obtained. It was determined by integration.

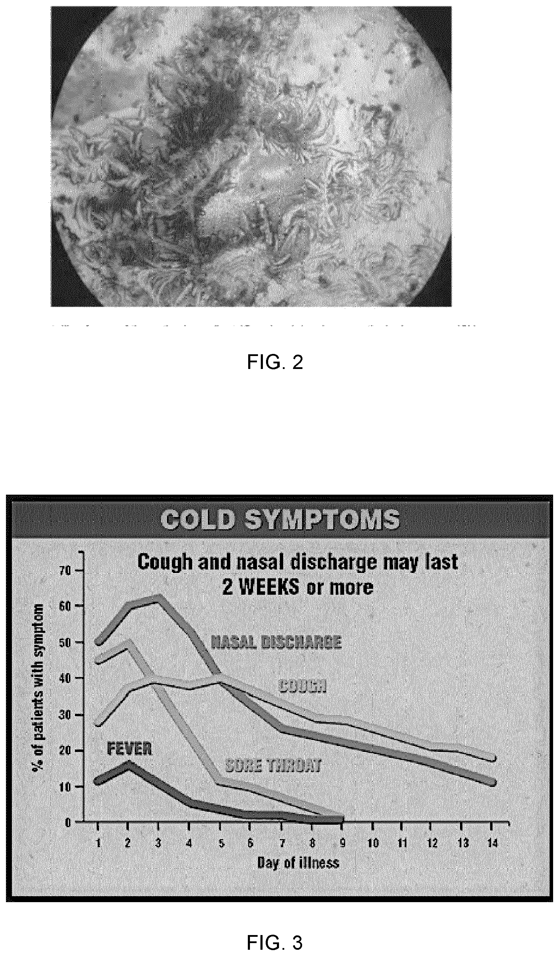

LC-MS using a waters UPLC/MS equipped with an Electrospray ion source was also carried out. The sample was dissolved in deionized water. Negative electrospray: m/z 387 [lactose+HCOO]⁻, m/z 341 [lactose-H]⁻, m/z 683 peak base [2 lactose-H]-, m/z 191 [Citric acid-H]-, m/z 161 loss of C₆H₁₂O₆ (180Da) from (lactose-H), m/z 179 loss of C₆H₁₀O₅ (162Da) from (lactose-H). Positive electrospray: m/z 360 peak base [lactose+NH₄]⁺, m/z 343 [lactose-H]+, m/z 325 loss of H₂O from [lactose-H], m/z 163 loss of C₆H₁₂O₆ (180Da) from (lactose-H), m/z 145 loss of H₂O (18Da) from m/z 163. FIG. 1 shows the X-ray diffractogram of the combination of the invention. FIG. 2 shows the crystal form of the combination under an optical microscope 40X.

### Example 2: Effect of the combination of the invention to treat cold and flu

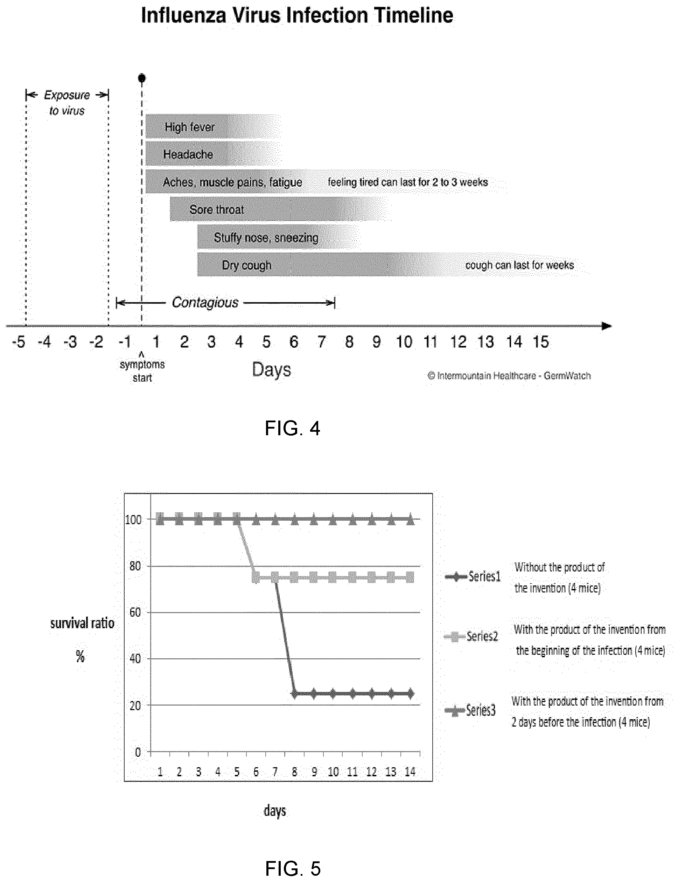

Knowing through official data, what is the average duration of the different symptoms that accompany both colds and flu (see FIG. 3 and FIG. 4), as it is about offering objective data, it was considered that personal appraisals about the intensity of the symptoms should be avoided, and it was determined that the only objective data could be the total disappearance of the different symptoms (although there really is a significant decrease in the intensity of the symptoms).

The results presented below are the result of tests carried out in the flu / cold season between autumn 2018 - winter 2019.

Each person was given the equivalent of two full treatments (5 days each) to consider that they could suffer more than flu or cold throughout the season, and on the other hand could be individuals who need more day's treatment than others. Each treatment consisted of 15 doses of the preparation, and each dose was administered orally every 8 hours. Each dose containing 600 mg of active ingredient that were presented in capsules.

The small size of the capsules did not report any type of problem to take them. The doses were delivered at the beginning of the cold season, so that people were available of the preparation at home in order to start taking it as soon as possible after the onset of symptoms.

The two complete treatments were provided to 150 individuals, requesting as the only consideration, the completion of a questionnaire that referred to multiple aspects related to the duration and intensity of the symptoms suffered. The data are shown in Table 1 below.

**Table 1: Comparison of the duration of symptoms by using the combination of the**

| | Average value in hours | Minimum value recorded | Maximum value recorded |
|---|---|---|---|
| Headaches | 15.08 | 2 | 24 |
| Fatigue | 24.92 | 5 | 24 |
| Fever | 12.60 | 8 | 24 |
| General discomfort | 24.69 | 5 | 48 |
| Myalgias-bone pain | 33 | 12 | 48 |
| Nasal congestion-sneezing | 19.22 | 6 | 48 |
| Cough | 42.90 | 6 | 168 |
| Sore throat | 29.09 | 6 | 52 |
| Vomiting diarrheal | None | | |

### invention until total disappearance in colds

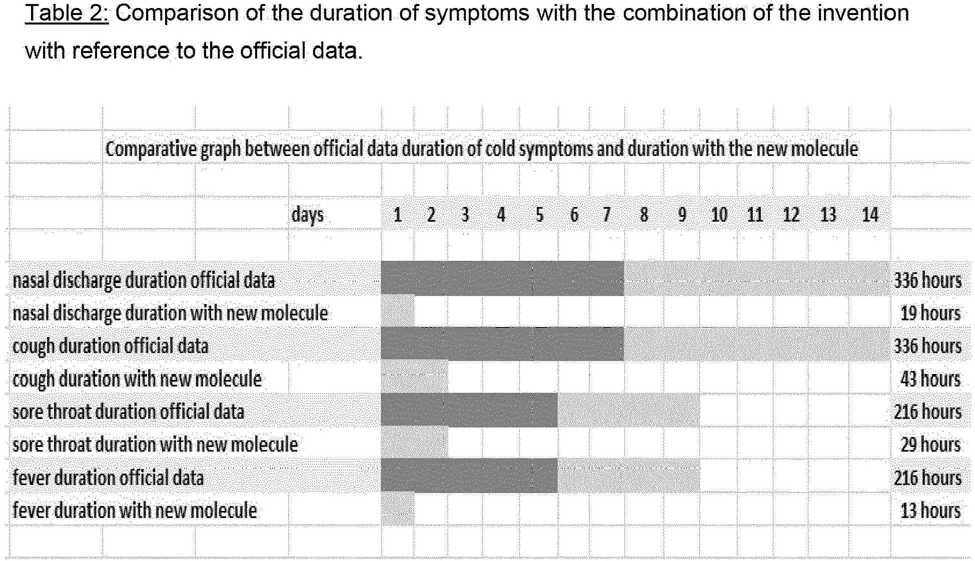

**Table 3 shows the duration of symptoms with the combination of the invention until its total disappearance in flu.**

| | Average value in hours | Minimum value recorded | Maximum value recorded |
|---|---|---|---|
| Headaches | 15.95 | 3 | 24 |
| Fatigue | 31.10 | 3 | 96 |
| Fever | 19.11 | 3 | 48 |
| General discomfort | 42.70 | 3 | 72 |
| Myalgias-bone pain | 27.11 | 3 | 72 |
| Nasal congestion-sneezing | 32.86 | 3 | 72 |
| Cough | 62.11 | 3 | 168 |
| Sore throat | 33.33 | 6 | 72 |
| Vomiting diarrheal | 26.40 | 24 | 36 |

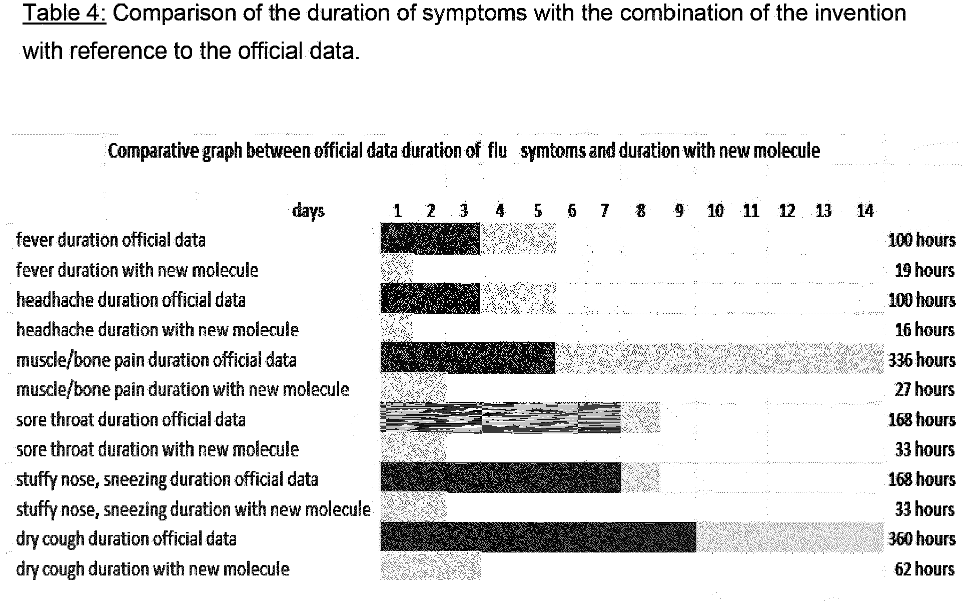

The results show a spectacular decrease in intensity and duration of the symptoms.

### Example 3: Effect of the product of the present invention to treat herpes simplex-1 virus

A preclinical in vivo study was carried out with mice infected with herpes simplex-1 virus (With a control group also infected but without treatment) at the Veterinary Faculty of the Autonomous University of Barcelona.The objective was to evaluate the effect of product of the present invention in mice infected with herpes simplex virus 1 (HSV-1).

Methodology: 6 groups of mice of the CD-1 strain were infected with HSV-1. One group was treated with the product of the present invention, and the rest served as control. In order to evaluate the effect of treatment on immune activity, the following variables were compared: mortality rate, serum expression of immune mediators (IFN and TNF-alpha) and immunoglobulins, NK cell activity and cytotoxic lymphocytes (CD8+) and anatomopathological study.

The results show that both under infection and non-infection conditions, significantly stimulated the secretion of pro-inflammatory cytokines. The stimulation determines an increase in activity of the phagocytic cells of the immune system: macrophages, neutrophils, and dendritic cells; and of the different classes of T lymphocytes: cytotoxic T lymphocytes (or CD8 + lymphocytes), helper T lymphocytes (or CD4 + or helper lymphocytes), regulatory T lymphocytes, as well as NK cells.

It influences the activity of the immune system in its unspecific response, stimulation the production of interferon y (+78.94%), reducing mobilization of neutrophils and holding constant the lymphocyte populations during infection.

The interferon y in viral infections functions as the main factor that decides whether the immune reaction is inclined towards a biochemical and cellular type reaction or towards an antibody-mediated immune reaction (the latter often leads to the chronification of the process, since a large part of the virus is located inside the cell, escaping to the action of the antibody). Then the biochemical and cellular mediated reaction (generally cytokines and cells) tends to be better. Thus, an increase in the secretion of interferon y (+78.94) is a tremendously positive factor. Reducing the mobilization of neutrophils is also a very positive factor as it controls (reduces) the inflammatory process. Keeping leukocyte counts constant is also good, since in general, during the infection and due to the fight against the virus, leukocyte populations are reduced, and this reduces the effectiveness of the immune response.

The assay with mice at the Veterinary Faculty, CAV 1 does not induce clinically or anatomopathologically visible adverse effects in mice".

A second assay with mice infected with herpes simplex-1 virus was carried out. The route of infection was intranasal. The mice were purchased at the UIB animal facility (University of the Balearic Islands).

The results can be seen in the following table and FIG. 5.

| | Survival ratio% | | | | | | | | | | | | | | |
|---|---|---|---|---|---|---|---|---|---|---|---|---|---|---|---|
| Without product (4 mice) | 100 | 100 | 100 | 100 | 100 | 75 | 75 | 25 | 25 | | 25 | 25 | 25 | 25 | 25 |
| With product from beqinninq infection (4 mice) | 100 | 100 | 100 | 100 | 100 | 75 | 75 | 75 | 75 | | 75 | 75 | 75 | 75 | 75 |
| With product from 2 days before the infection (4 mice) | 100 | 100 | 100 | 100 | 100 | 100 | 100 | 100 | 100 | | 100 | 100 | 100 | 100 | 100 |
| | 1 | 2 | 3 | 4 | 5 | 6 | 7 | 8 | 9 | 10 | | 11 | 12 | 13 | 14 |
| | days | | | | | | | | | | | | | | |

FIG. 5 shows the infective dose. This figure shows the influence of the product of the present invention on the activity of the immune system in its unspecific response, reducing mobilization of neutrophils and holding constant the lymphocyte populations during infection. In mice artificially infected with herpes simplex virus-1 (HSV-1) it was clearly observed that the product of the present invention increased their survival rate in a statistically significant way.

### Example 4: Evaluation of a product according to the invention on hPBMC assay

The product was tested in human PBMC at the following concentration: from 5ug/ml to 5mg/ml measuring the cell proliferation and cytokines release involved in the common inflammatory response (i.e., NF-Kb pathway).

The Concentration tested was based on maximal solubility measured in water (50mg/ml) diluted 1:10 in culture medium. No precipitation was detected under this condition. Cell proliferation was not observed after 24h from treatment.

Experimental protocol: 1) Whole blood from 3 healthy donors. non-smokers male, 2) Human Peripheral Mononuclear Cells (hPBMCs) was extracted, 3) Cell number and cellular population fractionation calculated through DASIT, 4) Cells plated at the density of 7'500'000 cells/mL, 5) Compound X was dissolved in milliQ water diluted in plate and added at 7 different concentrations ranging from 5ng to 5mg/mL, 6) Compound addition: 10mL in 90mL medium, 7) After 24h. medium was harvested and stored at -80°, 8) Cytokines release was evaluated via 4-plex Milliplex technology.

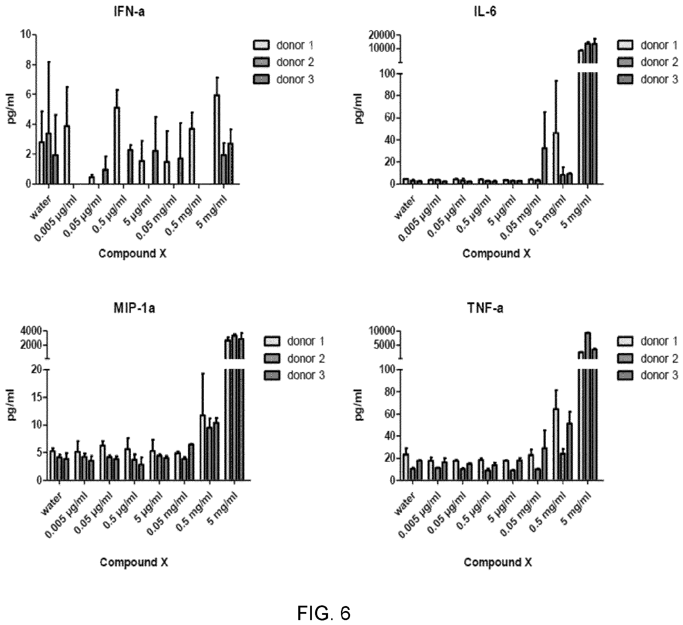

In another in vitro preclinical study, conducted by the German CRO Evotec, it was determined that the activity of the new molecule in front human not infected PBMC (Peripheral blood mononuclear cell) consisted of an increase of IL-6, MIP-1 and TNF-α after the concentration of 0.5mg/ml. IFN-α was not modulated at any concentration tested. The results of the cytokines release assay are included in FIG.6.

In an in vitro preclinical study, carried out by the German CRO Evotec, it was determined that the activity of the new product according to the present invention in front human not infected PBMC (Peripheral blood mononuclear cell) consisted of an increase of IL-6, MIP-1 and TNF-α. These preclinical studies showed that the new molecule, both under infection and non-infection conditions, significantly stimulated the secretion of pro-inflammatory cytokines. This stimulation determined an increase in activity, on the one hand of the phagocytic cells of the immune system: macrophages, neutrophils, and dendritic cells. And on the other hand of the different classes of T lymphocytes: cytotoxic T lymphocytes (or CD8 + lymphocytes), helper T lymphocytes (or CD4 + or helper lymphocytes), regulatory T lymphocytes, as well as NK cells. Whose sequential and coordinated action would explain the effects observed in the cure of different viral diseases.

### Example 5: in vivo experiment in dogs with parvovirus and in dogs with respiratory symptoms that did not respond to antibiotic therapy

Clinical trials were then carried out in dogs with parvovirus and in dogs with respiratory symptoms that did not respond to antibiotic therapy (suspected viral infection).

Canine Parvovirus. Parvovirus is a clinical disease associated with a mortality of 30-40%. With 40 cases of puppies' sick with canine parvovirus. Standard treatment is symptomatic. There is no specific treatment against the causative virus.

Infectious enteritis in dogs, especially in puppies, is the most common, highly contagious and often lethal disease caused by parvo virus infection (PVC), which represents a very serious problem worldwide in the pet world. The incubation period ranges from 3 to 5 days, and the characteristic symptoms of the disease include hemorrhagic diarrhea, vomiting, lack of appetite, depression, acute dehydration, collapse and death. The disease usually starts abruptly and affects especially puppies, old dogs, immunocompromised animals, and certain breeds that are genetically susceptible. At least 80% of deaths occur within the first 5 days, and as a general rule 10 to 15 days are necessary to achieve full recovery of the animals.

Prophylaxis is carried out by means of different types of vaccines, generally very effective, but with a relatively high immunization failure rate.

At present, there are no known specific antiviral treatments against PVC infection, although there are supportive treatments, consisting mainly of intravenous fluids and electrolytes, relief of vomiting and pain associated with intestinal spasms with antiemetic medication and lytic spasm, and application of antibiotics to prevent secondary bacterial complications. Initiating treatment in the early stages of the disease, mortality is between 20-50 % (the mortality rate is very much conditioned to the socioeconomic level of the clinic's clientele, since as a general rule the higher the level, the earlier the visit to the clinic and thus the earlier the start of treatment, which greatly increases the survival rate).

### Inclusion criteria

- Minimum age 30 days, acceptance of both sexes, acceptance of vaccinated and unvaccinated animals.
- Clinical diagnosis of PVC, with obligatory presence of bloody diarrhea for at least 1 day, and presence of two other symptoms among the following: vomiting, dehydration, anorexia, fever, sensory depression.
- Presence of PVC amphigens in stool, using the IDEXX laboratories detection test.

### Supportive treatment (common in both groups)

- Hospitalization in consultation with the criterion of discharge from the moment when food intake has normalized. This stage is usually 1 or 2 days after the majority (but not total) disappearance of clinical symptoms.
- Supportive treatment based on intravenous fluid therapy (Braun brand Ringer's lactate) with dosage based on the degree of dehydration, on a mandatory basis. Administration of antiemetics and spasmolytics if necessary. Administration of antibiotics (penicillin + streptomycin) on a mandatory basis.
- No vaccination, no application of immunomodulators.

### Treatment with the product of the present invention:

All subjected to the same basic treatment. Twenty of them were given the new product of the present invention and the other 20 were not.

Control group (without incorporation of the active ingredient to the treatment), and problem group (with incorporation of the active ingredient to the treatment, at a dose of 1 mg/kilo of weight every 8 hours subcutaneously, although on Saturdays and Sundays it was administered mixed with the serum intravenously).

### Chronology

The trial was carried out in the period from October 2000 to May 2002, starting in both clinics on the same date, and finishing the data collection with some months of difference, since we agreed to evaluate the first 20 cases received. This approximate coincidence in time was considered important since, depending on the different years, the virulence of the PVC strains varies considerably. Once the data collection was finished, we proceeded to its elaboration and evaluation, proceeding to the preparation of 2 graphs, one referring to the evolution of the clinical status and the other to the survival rate. The data were not subjected to statistical analysis since we do not have specific knowledge in this area, and we only intended to carry out an approach to the problem. The graphs obtained were as follows.

### Clinical evolution

To determine this section, the presence of 5 symptoms was chosen: Vomiting, bloody diarrhea, dehydration, sensory clouding, and refusal to eat. These symptoms were rated from 0 to 3 according to their severity and the clinical assessment was established by the sum of the individual values reached for each symptom.

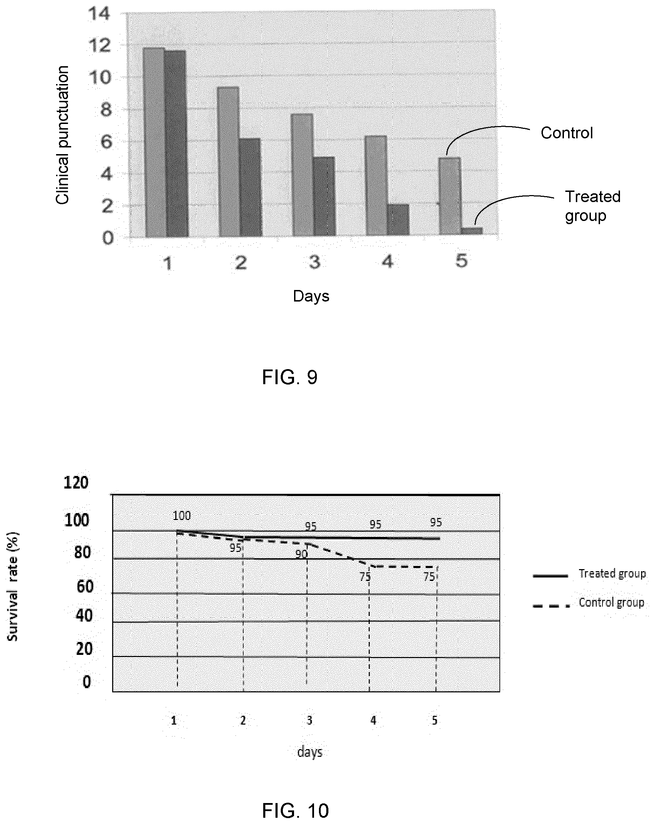

With respect to the clinical evolution, it should be noted that although on the first day the scores obtained are practically identical (as expected), on the 2^{nd} day there is a significant improvement in the group in whose treatment the active ingredient was included with respect to the control group (without inclusion of the active ingredient). This difference remained more or less stable throughout the 3^{rd} day and became much more pronounced on the 4^{th} and 5^{th} days. Evidently, this improvement in the clinical condition largely determined the survival rate (see FIG. 9).

Regarding the survival rate, it should be noted that in the animals of the control group, it was 75%, which indicates that there was a mortality of 25%, a rate that can be considered within normal parameters, since with a prompt application of the treatment and with hospitalization, this rate is usually around 25-30%. As for the animals in the problem group (in whose treatment the active ingredient was included), the survival rate reached 95%, since mortality was only 5%. This indicates that in the experience carried out, the survival rate of the animals treated with the inclusion of the active ingredient was multiplied by 5 with respect to those not treated with the active ingredient (see FIG. 10).

Canine enteric Coronavirus. Although this disease produces a reduced % of deaths,mthis disease normally occurs in acute form for 4-5 days, mainly producing diarrhea, tremors, fever, loss of appetite and vomiting. Sometimes some of these symptoms can last for 3 or 4 weeks. With the administration of the new product of the present invention, the total healing period could be established in 1-2 days. Standard treatment is symptomatic. There is no specific treatment against the causative virus.

The administration of the product of the present invention allowed to diminish mortality up to 5% and reduce the symptomatology associated, increasing the quality of life of the animals according to a clinical evaluation scale.

The therapeutic effect of the product of the present inventions against the viral respiratory disease in animals was immediate and far superior to traditional treatments.

### Conclusion of the results of Example 1-5

The data obtained in animal models of viral diseases in various species (canids, felids and murids), in different organic systems (respiratory, gastrointestinal and nervous) and against very diverse families of viruses (influenza, herpes, parvovirus, papilloma and coronavirus), suggest a broad spectrum of action and a powerful therapeutic effect. The effect of the product of the present invention was mainly manifested in the great increase in the survival rate in infected animals (between 20% and 75%) and in a significant improvement of the observed symptoms. Its wide and powerful therapeutic spectrum support in the treatment of various diseases of viral origin in the human species.

### Example 6: Evaluation of the product of the present invention in the treatment of plantar wart

The product of the present invention was also administered to several patients having human papillomavirus. The human papilloma virus comprises a number of viral variants causing multiple disorders. Some of them like warts, although of little clinical importance if they produce disorder of psychological or aesthetic nature, others like plantar papilloma are most important because they cause severe pain and discomfort, others such as genital papilloma are of great significance especially in women as it contributes decisively to the development of cervical cancer way (this cancer today is considered placed in second place after breast as a cause of cancer death in women).

A case that is considered very significant corresponds to a plantar papilloma very advanced stage. It corresponds to an 18-year-old girl. The case was difficult because the process had begun 5 years ago, being initially misdiagnosed. In the last year there had been a worsening. The last treatment consisted of monthly cryonics, accompanied by a scraping back. Also, they applied twice daily based abrasive salicylic acid solutions, more scraping. But after almost a year following this treatment, the process far from abating had worsened considerably, increasing its size and the discomfort and pain caused, which caused severe functional disorders, difficulty in walking. There had been an anatomical deformation in the foot, and psychological type disorders, as the patient had suppressed any outdoor activity that would involve your shoes, because the aspect of the foot was really unpleasant. The treatment with the product of the present invention was carried out in 2 batches of 15 days each, with a break in between 15 days. This interruption was carried out for 15 days of starting treatment. The treatment was resumed administered over a period of another 15 days, after which it was interrupted as he had achieved complete healing. The development process was very gradually healing, because the 3-4 days of starting treatment early improvements already were observed, and these were happening gradually until complete healing. In the period of 15 days off, no improvement was apparent but not regression to earlier stages. Since healing in April 2003, there has been no recurrence.

Another case corresponds to a 16 year old girl having plantar papilloma and having carried out a previous treatment (monthly cryogenization and daily defoliant ointment). At two months of initiation treatment was noted the uselessness of this as the papilloma was increasing in size and causing more discomfort. The treatment with the product of the present invention began on September 28, 2004, and was completed on October 10, 2004, with total healing. No recurrences have occurred.

Another case was the one of 45 years old patient. The process began in June 2005 with little discomfort in the area defined planting the right foot; the discomfort was increasing, and the presence of a papilloma was confirmed by a podiatrist in September 2005. This applied a treatment based topical application of acid, which lasted for a month and a half, without apparent results since the process was worsen. At the end-October 2005 the existence of papilloma was confirmed. The dermatologist established a treatment consisting in taking vitamin A (Oranzinc), topical application of glutaraldehyde and cryogenic lesions by topical application of liquid nitrogen (4 sessions with nitrogen were conducted). The treatment lasted for approximately 45 days (late November 2005). The new treatment failure, and dermatologist established the possibility of chemotherapy or radiotherapy sessions, something which was rejected by the patient. This patient also suffered chronically from an autoimmune disease called Cristopatia Interstitial consisting of immune system attacks the internal lining of the urinary bladder, which suffers frequent episodes and has restricted the taking of many foods and many drugs. Treatment with the product of the present invention began 2006-01-16 , and lasted over just 10 days, the third day of starting papilloma treatment, started necrotic and reduce pain significantly. The patient finding the 10 days started to come off the milder papilloma, he decided to stop treatment and be on the lookout for its evolution. It did not reinstitute treatment, and important issue, no episodes of autoimmune process was observed.

The results show that there seem to be two types of mechanism of action, in case 1 the lesions gradually disappear. In case 2 a necrosis of the lesions is clearly seen, most likely due to an attack of cytotoxic cells.

### Example 7: Evaluation of the product of the present invention in the treatment of herpes zoster

The product was administered to several patients and provided very good results.

One of the patients was a 52-year-old woman who regularly consumed the active ingredient in curing colds and flu. She was diagnosed with herpes zoster. The injury suffered was important as it was located on the right costal area and had a length of about 20 centimeters in diameter. Administration of the product of the present invention to the usual doses began, with results considered extraordinary. After three days of starting treatment itching and pain had disappeared totally, besides producing an evolution of the vesicles which had become scabs. At 5 days of starting treatment scabs fell, it being completed treatment.

Another patient was a woman 91-year-old suffering herpes zoster in the sacral area of about 10 centimeters in diameter. the Acyclovir did not produce a positive result. The product of the present invention was then administered. It took almost 3 weeks to get complete healing, albeit from 4-5 days, the improvement was significant. After these two years, there been no recurrence or has performed post-herpetic pain.

### Example 8: Evaluation of the formulation of the invention in animals with cancer

The formulation was tested in 2 cases: the first case was a Canine cutaneous epitheliotropic T-cell lymphoma in Poi a boxer dog 10 years old at the start of treatment, and the second one was a mammary adenocarcinoma to Chispa, a mongrel bitch, that he had at the start of treatment 6 years old.

Canine cutaneous epitheliotropic T-cell lymphoma: In the dog cutaneous epitheliotropic T-cell lymphoma, represents 3-8% of all lymphomas. Usually, it occurs after 10 years. When presented plate, as is the case presented, there may be solitary or multiple, erythematous, pruritic and more or less exfoliative lesions, and often ulcerated. The tumours are malignant because of its ease in metastasize; process develops very quickly causing the animal's death so explosive. Currently no effective treatment is known. The only treatment is used occasionally systemic chemotherapy with a combination of vincristine, and prednisolone, this causes a temporary remission of symptoms, but only manages to prolong the life of the animal for up to 3-4 months.

The animal treated with the active ingredient, corresponded to a male boxer dog 10 years old at the start of therapy. At that time the dog wearing three years undergoing treatment and continued control against leishmaniasis. In one of these periodic checks which was caused by a severe deterioration of the condition of the animal, a lip injury was detected which was diagnosed as a lymphoma. The proposed chemotherapy treatment (surgery and radiotherapy) would only prolong the life of the animal for a few months. To this it should be added the general dog was miserable, with a pronounced apathy, anorexia and cachexia, compounded by immunodeficiency caused by leishmaniasis. In this perspective chemotherapy was discarded.

The medication leishmaniasis was continued. The administration of the formulation of the present invention was the only active ingredient as a single therapy against lymphoma. Against all forecast, all forecast, three weeks of initiation of treatment (day 20-11-2005) external injury lymphoma disappeared completely, and 2 months after the animal fully recover their physical and emotional way. After 21 days of starting treatment the lesion had completely disappeared. The dog died of natural causes (not attributable to lymphoma) on June 19, 2007, so his life lasted for 19 months, compared with an initial life expectancy of only 3-4 months if he had made the chemotherapy.

Mammary carcinoma: Mammary tumours are the most common types of tumours in bitches. Half of them is benign and malign the other half. Among malignant tumours mixed cell type are most; followed by carcinomas (preferably adenocarcinomas) and are less frequent sarcomas. Carcinomas and sarcomas are preferably capable of causing metastases in the lymph nodes in the area and in different organs, primarily in the lung. Treatment includes surgery, chemotherapy and radiotherapy depending on cases. The prognosis varies depending on the malignancy of Tumour and when the treatment is initiated.

Chispa bitch whose case is presented, was born on 15/11/2000. She was diagnosed with mammary tumour with ganglionar involvement (with an approximate size of 2.5 cm. long by 1.5 cm. wide) in May 2005. Pathological analysis of the tumor which resulted in the diagnosis of adenocarcinoma. In the same month of May underwent surgery, proceeding to resection of the tumor and the corresponding inguinal node. In June 2006 the dog was brought back to the consultation and tumor recurrence was verified, which at that time had a size of 3 cm. long by 1.5 cm wide by 1 cm deep. Tumor growth to reach this size took place about a month. In such cases of recurrence, also having such rapid growth, life expectancy is usually up to 2-3 months as inescapably accompanied by metastases in internal organs.

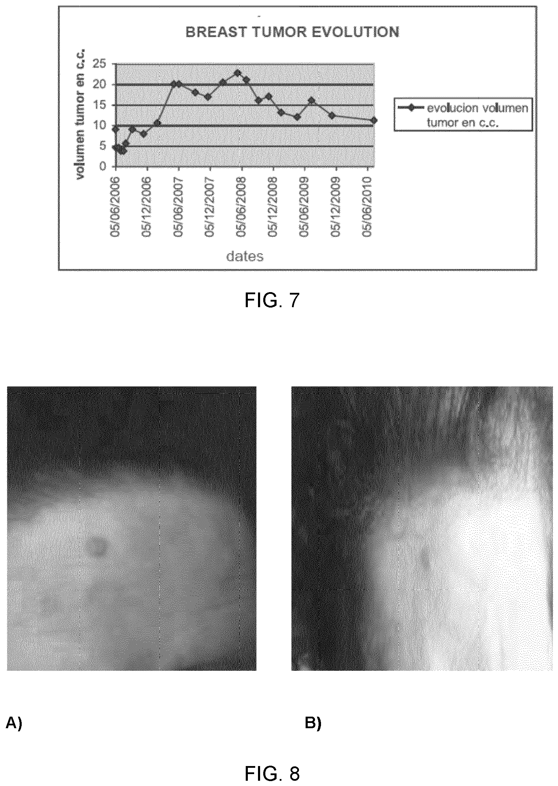

Treatment with the formulation according to the invention was tested starting the day 08/06/2006 treatment, according to common guidelines and dose of active ingredient FIG. 7 series shows the evolution of the tumor: The observations were carried out until 05/06/2010. At that time, it was decided to finish the treatment because the tumor had for years been practically stabilized. Tumor progression is seen in FIG. 8.

The bitch died in October 2010 of sudden death; A necropsy showed that the main cause of death was cardiac hyperplasia that had no relation to the neoplastic process. No metastases in organs or lymph node involvement were observed. A sample of the tumor was scanned. The results showed that the malignant nature of the tumor had remained, but the growth stopped, and metastases were avoided. Thus, the chronicity of the process was achieved unlike the recurrences that generally occur after practicing surgery because at that stage the tumor, present explosive growth, which are always accompanied by metastasis to other vital organs and expectation of survival not usually it extends beyond 2 or 3 months.

The control of the tumor was attributable to the formulation of the invention and made it possible that the survival process lasted for 4 years and 2 months. A factor to note, is that both dogs suffering from canine leishmaniasis: one of the characteristics of this disease is that it compromises greatly the immune system, since reproduction of leishmania is carried out on macrophages, which are destroyed when released these. That is, both dogs started from an immunosuppressed initial situation.

### Example 9: Evaluation of the formulation of the invention in humans

Melanoma: The patient had a mole situated before the right ear for many years, The mole suffered a malignant disruption. As growth mole was very sudden, clear and fast, itched and had started bleeding. After 3 days of administering the formulation of the present invention, the mole began as to project to the outside and dry up, at 10 days it was completely dry and detached.

Basal cell carcinoma: Female 92 years with a history of pathologic diagnosis of basal cell carcinoma with subsequent surgical removal. Appearance of new tumor after about 2 years. The treatment consisted of the administration of the active ingredient orally, 2 times a day and the application of the active ingredient powder on the injury (the injury was covered with the active ingredient and covered everything with a breathable dressing, the process was repeated daily). FIG 8 shows the results.

### Example 10: Results of the evaluation of the product of the invention in several diseases

There are other viral diseases successfully treated. In these cases, the good results reported are based on communications made by users who had the product to treat mainly colds and flu and that, given the alleged presentation of other diseases of viral etiology, their effectiveness was tested for the following diseases:
a) Infectious mononucleosis: caused by the Epstein-Barr virus causes fever, sore throat, and swollen lymph nodes in the neck. (+ than 15 cases)
b) Viral Pneumonia: caused by several different viruses, including influenza, parainfluenza, respiratory syncytial virus, adenovirus, coronavirus and hantavirus (+ than 200 cases).
c) Stomach flu (viral gastroenteritis): caused by several different viruses, such as norovirus, rotavirus, enteric adenovirus and astrovirus, cause diarrhea and vomiting. (+ than 200 cases)
d) Viral conjunctivitis: caused mainly by the adenovirus causes red eyes, itching and watery eyes. (+ than 15 cases).
e) Mumps: caused by the mumps virus, causes fever and swelling of the parotid glands. (+ than 15 cases in dogs).
f) Cytomegalovirus: caused by cytomegalovirus, causes a symptomatology similar to mononucleosis, with fever and enlargement of the lymphatic chain of the neck. (+ than 15 cases).
g) Varicella: The virus that causes Herpes Zoster (VZV) belongs to the genus Varicella Virus of the family of Herpes. VZV in its primary infection causes chickenpox, a very frequent infantile exanthematous infection (+ than 15 cases).
h) Significant improvement in the discomfort of Premenstrual Syndrome: reported from women with this problem that have improved their discomfort in a percentage that they estimated between 50-85%. This is based on a subjective criterion.
i) Resolution of chronic inflammation. Reported from a man who throughout a year and a half developed a chronic tendinitis of both Achilles tendons. The man was cured after taking the active ingredient for three months.
j) Improvement of physical performance. Reported from a swimmer who improved his swimming performance reducing his times after taking the product of the present invention.

### Citation List

### Non-Patent Literature

- Alhazmi et al., "Medicinal Plants and Isolated Molecules Demonstrating Immunomodulation Activity as Potential Alternative Therapies for Viral Diseases Including COVID-19 Natural Immunomodulators for Viral Diseases" 2021, vol.12 (https://doi.org/10.3389/fimmu.2021.637553).
- T Nagai et al, in "In vivo anti-influenza virus activity of Kampo (Japanese herbal) medicine "Sho-Seiryu-to -stimulation of mucosal immune system and effect on allergic pulmonary inflammation model mice" Immunopharmacology and Immunotoxicology, 1998, vol. 20, pp. 267-281.
- Yao et al.; "a-Lactose Improves the Survival of Septic Mice by Blockade of TIM-3 Signaling to Prevent NKT Cell Apoptosis and Attenuate Cytokine Storm"; Shock (Augusta, Ga. 2017, vol. 47(3), pp. 337-345).
- R. Chowdhury et al.; in " Effect of citric acid, avilamycin, and their combination on the performance, tibia ash, and immune status of broilers." Poult. Sci., 2009, vol. 88, pp. 1616-1622.
- Abdel-Fattah, S. A. et al., "Thyroid activity of broiler chicks fed supplemental organic acids.", Int. J. Poult. Sci, 2008, vol. 7, pp. 215-222).

## Claims

1. A product which comprises a crystallized mixture of two active ingredients, wherein the crystallized mixture is a mixture in which the two active ingredients have crystallized together; and wherein the active ingredients are:
a) lactose or a pharmaceutically acceptable salt thereof, and
b) citric acid or a pharmaceutically acceptable salt thereof,
in a weight ratio in the crystallized mixture from 89:11 to 69:31, expressed with respect to lactose:citric acid in free form.

2. The product according to claim 1, which comprises lactose in free form.

3. The product according to any of the claims 1-2, which comprises citric acid in free form.

4. The product of any of the claims 1-3, wherein the weight ratio lactose:citric acid in the crystallized mixture is from 80:20 to 70:30.

5. The product according to claim 4, wherein the weight ratio lactose:citric acid in the crystallized mixture is 77:23.

6. The product of any of the claims 1-5, **characterized by** having an X-ray diffractogram that comprises characteristic peaks at approximately 12.5, 16.4, 19.1, 19.6, 20.9, 21.2, and 23.8 degrees 2 theta at a Cu-K_{α}1 radiation =1,54060 Å.

7. A process for preparing the product as defined in any of the claims 1-6, comprising the steps of:
a) mixing the lactose or the pharmaceutically acceptable salt thereof in water at an appropriate temperature to form a solution;
b) mixing the citric acid or the pharmaceutically acceptable salt thereof in water at an appropriate temperature to form a solution;
c) mixing the solution a) with the solution b) in a proportion such that the weight ratio lactose: citric acid is from 89:11 to 69:31; and d) isolating the compound thus obtained.

8. A food supplement comprising an immunomodulatory effective amount of the product according to any of the claims 1-6.

9. A pharmaceutical composition comprising a therapeutically effective amount of the product as defined in any of the claims 1-6, together with appropriate pharmaceutically excipients and/or carriers.

10. A product as defined in any of the claims 1-6, for use as a medicament.

11. A product as defined in any of the claims 1-6, for use for modulating the immune system.

12. A product as defined in any of the claims 1-6, for use in the prevention and/or treatment of: a disease or condition through the modulation of the immune system, wherein the disease or condition is selected from the group consisting of influenza, common cold; herpes simplex, herpes zoster, infections caused by the human papilloma virus, stomach flu, viral pneumonia, mononucleosis, viral conjunctivitis, mumps, cytomegalovirus, varicella, smallpox, respiratory syncytial virus, cancer, hepatitis B, hepatitis C, and chronic inflammation.

13. The product for use according to claim 12, wherein the disease or condition is influenza or common cold.

14. The product for use according to claim 12, wherein the the infections caused by the human papilloma virus are selected from the group consisting of plantar papilloma, warts, genital papilloma in both men and women, anal papilloma, and cervical papilloma in women. Cervical papilloma in women often leads to cervical cancer. In another particular embodiment, the infections caused by the papilloma virus are selected from plantar papilloma and warts.

15. Use of the product as defined in any of the claims 1-6, for improving the discomfort of Premenstrual Syndrome or for improving physical performance.
